(19) Europäisches Patentamt / European Patent Office / Office européen des brevets

(11) **EP 4 512 360 A1**

(12) **EUROPEAN PATENT APPLICATION**

(43) Date of publication:
**26.02.2025 Bulletin 2025/09**

(21) Application number: **24153415.5**

(22) Date of filing: **23.01.2024**

(51) International Patent Classification (IPC):
**A61B 90/00** $^{(2016.01)}$ **A61B 34/10** $^{(2016.01)}$

(52) Cooperative Patent Classification (CPC):
**A61B 90/36; A61B 34/10; A61B 90/13;**
A61B 2034/107; A61B 2090/365; A61B 2090/372;
A61B 2090/3762

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **HOPFGARTNER, Christian**
**90763 Fürth (DE)**
• **SÜHLING, Michael**
**91052 Erlangen (DE)**
• **WIMMER, Andreas**
**91301 Forchheim (DE)**

(74) Representative: **Siemens Healthineers Patent Attorneys**
**Postfach 22 16 34**
**80506 München (DE)**

(54) **CAMERA-BASED GUIDANCE FOR NEEDLE INTERVENTION**

(57)  A method for generating augmented camera image data (ACID) for guiding a needle intervention is described. According to the method for determining information for guiding a needle intervention, medical image data (MID) of an examination portion of a patient are received and also camera image data (CID) of the examination portion are received. Further, information concerning a planned insertion pose of the needle (1) are determined based on the received medical image data (MID), wherein coordinates ($q_{CT}$, $u_{CT}$, $v_{CT}$) assigned to the planned insertion pose of the needle (1) in a coordinate system ($KS_{CT}$) of the medical image data are (MID) determined. Furthermore, coordinates ($q_{2D}$, $u_{2D}$, $v_{2D}$, $q_{CAM}$, $u_{CAM}$, $v_{CAM}$) assigned to the planned insertion pose of the needle (1) in the camera coordinate system ($KS_{CAM}$) of the camera (CAM) are determined by transforming the coordinates ($q_{CT}$, $u_{CT}$, $v_{CT}$) assigned to the planned insertion pose from the medical image data coordinate system ($KS_{CT}$) into the camera coordinate system ($KS_{CAM}$). In the end, augmented camera image data (ACID) are generated based on the determined coordinates ($q_{2D}$, $u_{2D}$, $v_{2D}$, $q_{CAM}$, $u_{CAM}$, $v_{CAM}$) in the camera coordinate system ($KS_{CAM}$) and the received camera image data (CID). Further, a camera-based method for guiding a needle intervention is described. Also an intervention control device (100) is described. Furthermore, an intervention system (110) is described.

FIG 9    900

9.I — $P \rightarrow MID$

— MID

9.II —

— CID

9.III — $KS_{CT} \rightarrow q_{CT}, u_{CT}, v_{CT}$

CID — $q_{CT}, u_{CT}, v_{CT}$

9.IV —

CID — $q_{CAM}, u_{CAM}, v_{CAM}$

9.V — $q_{CAM}, u_{CAM}, v_{CAM} \rightarrow p_{2D}, u_{2D}, v_{2D}$

9.VI — $p_{2D}, u_{2D}, v_{2D} \rightarrow ACID$

**EP 4 512 360 A1**

Processed by Luminess, 75001 PARIS (FR)

**EP 4 512 360 A1**

**Description**

The invention relates to a method for generating augmented camera image data for guiding a needle intervention. The invention also concerns a camera-based method for guiding a needle intervention. Further, the invention refers to an intervention control device. Furthermore, the invention relates to an intervention system.

Medical imaging systems are also often used to guide needle interventions like biopsies. During preparation of the intervention, a needle path is planned based on a planning scan, e.g. a CT spiral scan. The planning consists of annotating a target point inside the body where the needle tip shall be positioned, and a needle insertion point on the skin surface. The needle path is planned such that sensitive structures like vessels, other organs, or bones are not harmed. The key challenge in intervention workflows is to transfer the needle path planned based on the medical data to the actual patient. In particular, the exact insertion point on the skin has to be located and the correct direction has to be applied when inserting the needle into the body.

For identification of the needle entry point on the patient's skin, the following workflow is most common: the z-position of the slice (the z-axis is the symmetry axis of the medical imaging system) in which the entry point is located is read from the image text of the planning scan. The patient table is positioned in a way that this z-position exactly lines up with the scan plane, which is marked by the gantry laser. In this way, the z-position for the injection point is located. To identify the x- and y-position within the z-plane, the distance on the skin from the central laser is measured within the planning image and is also measured on the actual patient with a ruler and marked on the skin. As an alternative for this measurement, a metal grid can be used which is taped to the skin before the planning scan and which has metal wires running parallel in z-direction.

The wires appear on the planning scan and can be used to identify the ideal injection point (e.g. between the 3rd and 4th wire from the left). When the injection point has been located and marked on the patient's skin using one of the two methods described above, the needle must be inserted into the skin with the correct angle as planned in the planning scan. The user views the planned needle path on an in-room monitor next to the patient and aligns the angle in which he injects the needle into patient's body with the angle of the planned needle path using an eye balling approach. If the needle path is only tilted within the x-y scan plane (in-plane intervention) this method works relatively well. However, if the needle is also tilted towards the z-axis (double angulated needle path) this is not easy to accomplish by eyeballing resulting in a decreased precision. As an alternative, few radiologists actually measure the angle of the needle to match the angle on the planned needle path.

It is therefore a task to provide a method and a system for guiding a needle intervention with more reliability than in prior art.

This task is accomplished by a method for generating augmented camera image data for guiding a needle intervention according to claim 1, by a camera-based method for guiding a needle intervention according to claim 11, by an intervention control device according to claim 12 and by an intervention system according to claim 13.

The method for generating augmented camera image data for guiding a needle intervention according to the invention comprises the step of receiving medical image data of an examination portion of a patient. The examination portion comprises a region of the body of a patient which is intended to be the object of a medical intervention performed using a needle. The medical image data are generated by a medical imaging system. The medical imaging system comprises an imaging technique for generating 3D medical images of the inside of the body of a patient. The medical imaging system preferably comprises an X-ray based imaging system. Most preferably, the medical imaging system comprises a computer tomography system. However, the medical imaging may alternatively comprise other modalities, e.g. a magnetic resonance or ultrasound imaging system. The medical image data preferably comprise 3D images. The medical image data provide information for determining and planning a pose of a needle for a planned intervention. In particular, the medical image data comprise information about the interior of the body of a patient and details of the examination portion such that sub-portions of the inner body which have to be avoided by the needle and sub-portions of the inner body which are intended to be drilled through by the needle are able to be identified and to be localized. The medical image data are assigned to a coordinate system of the medical image data. Such a medical image data coordinate system preferably comprises a 3D-coordinate system including the origin on a symmetry axis, in particular the z-axis of the medical imaging system.

Further, camera image data of the examination portion are received. The camera image data are generated by an observation camera or monitoring camera being arranged in the examination room for observing the patient lying on the patient table of the medical imaging system. Preferably, the camera comprises a 2D image camera. 2D images enable a complete overview of an insertion scenario. The camera image data are intended to be used for monitoring and guiding a needle intervention in an examination portion of a patient. Generally, the coordinate system of the camera, also named camera coordinate system, differs from the coordinate system of the medical imaging system.

Furthermore, information concerning a planned insertion pose of the needle is determined based on the received medical image data as already mentioned above. The information about the planned insertion pose of the needle comprises at least information about a planned position of the needle, in particular of a planned insertion position of the needle and an orientation of the needle while the needle is inserted. Further, the information concerning a planned insertion pose of the needle preferably comprises information about the planned end position of the tip of the needle and also preferred information about the planned end position of the head of the needle and further preferred information about the planned orientation of the needle at the end position.

For determining the mentioned information, coordinates assigned to the planned insertion pose of the needle in the medical image data coordinate system are determined based on the medical image data. In particular, the medical image data provide information about organs or bones or tissue or blood vessels to be targeted in the body of a patient using the needle and organs or bones or tissue or blood vessels to be avoided by the needle.

Furthermore, coordinates assigned to the planned insertion pose of the needle in the camera coordinate system of the camera are determined by transforming the coordinates assigned to the planned insertion pose of the needle from the medical image data coordinate system into the camera coordinate system. The coordinate system of the medical imaging system and the coordinate system of the camera differ from each other due to different positions of the origins of these different systems, due to different orientation of these systems and possibly due to a different geometry of these systems. Based on the medical image data, a planned pose of the needle is able to be determined. However, since the template for the needle is to be shown in the display of the camera, a transformation between the coordinate systems of these different systems has to be performed.

In the end, augmented camera image data are generated based on the determined coordinates in the camera coordinate system and the received camera image data by combining templates or marks based on the determined coordinates with the received camera image data.

Using a camera, the workflow step of transferring a planned needle path to the actual patient can be simplified significantly. Many modern medical imaging systems are already equipped with cameras for patient observation or for patient positioning and scan automation. Moreover, observation cameras are also frequently installed in examination rooms. The use of an already existing camera in the context of an imaging-guided medical intervention workflow, particularly for the initial placement of the needle, provides a precise placement of the needle based on a path planned using the medical imaging data. The display of the needle tip may help to save time and to avoid mistakes during the otherwise manual transfer of the medical imaging planning result to the skin of the patient. In addition, having a good indication of the planned needle orientation and stop position may help to avoid some control scans during the intervention to track the needle, thereby saving time and radiation dose. In contrast to alternative solutions, there is no need for additional resources, as already existing and installed hardware is used for observing the needle.

In the camera-based method for guiding a needle intervention according to the invention, the method for generating augmented camera image data for guiding a needle according to the invention is performed, wherein coordinates assigned to a planned insertion pose of the needle in a camera coordinate system of a camera are determined and augmented camera image data are generated based thereon. The augmented camera image data preferably comprise templates or marks as needle alignment guide for guiding an insertion of the needle. Preferably, the templates or marks comprise a guideline, preferably comprising at least one of the following signs: a dotted line, a solid line, a marking cross, a marking circle. Further, the augmented camera image data are displayed.

Hence, the needle and a planned pose of the needle are displayed in the camera image data based on the determined coordinates. The camera-based method for guiding a needle intervention according to the invention shares the advantages of the method for generating augmented camera image data for guiding a needle intervention.

The intervention control device according to the invention comprises a medical image data interface for receiving medical image data of an examination portion of a patient and a camera image data interface for receiving camera image data of the examination portion.

Further, the intervention control device according to the invention comprises a determination unit for determining information concerning a planned insertion pose of a needle based on the received medical image data. For determining information concerning a planned insertion pose of the needle, coordinates assigned to the insertion pose of the needle in the medical image data coordinate system are determined based on the medical image data.

Furthermore, the intervention control device according to the invention comprises a transformation unit for determining the coordinates assigned to the insertion pose of the needle in the camera coordinate system of the camera by transforming the coordinates assigned to the insertion pose in the medical image data coordinate system into the camera coordinate system.

The intervention control device also comprises a generation unit for generating augmented camera image data based on the determined coordinates in the camera coordinate system and based on the received camera image data.

The intervention control device according to the invention shares the advantages of the camera-based guidance method for guiding a needle intervention according to the invention.

The intervention system according to the invention comprises a medical imaging system, preferably a CT-system, for generating medical image data from an examination portion of a patient. Further, the intervention system comprises a camera for observing the patient and for acquiring camera image data from the patient and in particular from the examination portion of the patient. Furthermore, the intervention system includes a needle for an intervention.

The intervention system also comprises an intervention control device according to the invention for generating augmented camera image data based on medical image data from the medical imaging system and camera data from the camera and for controlling the intervention using the needle based on the augmented camera image data.

The intervention system further comprises a display for displaying the augmented camera image data generated by the intervention control device. In the augmented camera image data, the needle and a template or mark indicating a planned pose of the needle are depicted. Hence, the display is used for displaying the camera image data and for displaying a template of the planned pose of the needle such that a physician is enabled to follow the displayed template with the needle. The intervention system shares the advantages of the intervention control device according to the invention.

Some units or modules of the intervention control device mentioned above can be completely or partially realized as software modules running on a processor of a respective computing system, e.g. of a control device of a medical imaging system. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the inventive method for generating augmented camera image data for guiding a needle intervention, in particular the steps of receiving medical image data, of receiving camera image data, of determining information concerning a planned insertion pose of the needle, of determining coordinates assigned to the planned insertion pose of the needle in the camera coordinate system and of generating augmented camera image data, or the software-based steps of the camera-based method for guiding a needle intervention, in particular the above-mentioned steps of the method for generating augmented camera image data for guiding a needle intervention, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a harddisk or other transportable or permanently-installed carrier can serve to transport and/or to store the executable parts of the computer program product so that these can be read from a processor unit of a computing system. A processor unit can comprise one or more microprocessors or their equivalents.

The dependent claims and the following description each contain particularly advantageous embodiments and developments of the invention. In particular, the claims of one claim category can also be developed analogously to the dependent claims of another claim category. In addition, within the scope of the invention, the various features of different exemplary embodiments and claims can also be combined to form new exemplary embodiments.

In a preferred variant of the method for generating augmented camera image data for guiding a needle intervention according to the invention, the information concerning a planned insertion pose of the needle in the received medical image data comprises an insertion point of the needle. Advantageously, the planned insertion point is displayed as template such that a physician is enabled to guide the needle to the virtually marked insertion position visible on a display.

Assuming that the patient has not moved since the planning scan was performed, the needle insertion point $q_{CT}$ from the planning scan, i.e. the medical image data in the medical image data coordinate system, can be transferred to the camera image point $q_{CAM}$ in the 3D camera coordinate system using the scanner to camera transform $T_{CT,CAM}$:

$$q_{CAM} = T_{CT,CAM} \cdot q_{CT}. \qquad (1)$$

In case at the intervention treatment that table position is different to the planning scan position, an additional translation regarding the new table position (describing by the transformation $T_{TABLE}$) has to be applied while computing the point in camera coordinates:

$$q_{CAM} = T_{CT,CAM} \cdot T_{TABLE} \cdot q_{CT}. \qquad (2)$$

The needle insertion point in camera coordinates $q_{CAM}$ is then projected into the camera image data using the intrinsic calibration expressed through the projection $P_{CAM}$:

$$q_{2D} = P_{CAM} \cdot q_{CAM}. \qquad (3)$$

The intrinsic calibration of the camera consists of the focal length f, the principal point, and distortion coefficients. A visual marker like a dot is displayed as an overlay onto the camera image in the camera image data. The physician can then align the tip of the actual needle with the marker to insert the needle at the right position.

As discussed above, preferably, the sub-step of transforming the coordinates assigned to the insertion pose in the medical image data coordinate system into a 3D camera coordinate system comprises an application of a camera transform $T_{CT,CAM}$ to the insertion pose assigned to the medical image data coordinate system. Advantageously, the coordinates are transformed into the coordinate system of the camera which is used for observing the needle and in particular the insertion of the needle.

As also mentioned above, preferably, the sub-step of transforming the coordinates assigned to the insertion pose in the medical image data coordinate system into a 3D camera coordinate system comprises an application of a transformation $T_{TABLE}$ representing a new table position in case the table position is changed. Advantageously, a movement of the table is taken into account for the coordinates in the camera image.

In a preferred version of the method for generating augmented camera image data for guiding a needle intervention according to the invention, the camera transform $T_{CT,CAM}$ is determined based on an extrinsic calibration step. Advantageously, the camera transform $T_{CT,CAM}$ can be calculated based on extrinsic calibration parameters. Extrinsic parameters describe the position and orientation of a camera in space.

Preferably, the camera image data comprise 2D camera image data as discussed later. Advantageously, 2D image data can be simply acquired and easily perceived by a physician, wherein the physician gets a complete overview of a needle insertion scenario.

Preferably, the information concerning a planned insertion pose of the needle based on the received medical image data comprises a needle orientation for the needle intervention and the step of determining information concerning an insertion pose of the needle in the received medical image data comprises at least one of the following sub-steps, preferably more than one of the following sub-steps, most preferably all of the following sub-steps, of determining:

- an end point of the needle at the start of the insertion based on a target needle tip position,
- a planned needle direction
  and
- the length of the needle.

Hence, the augmented-reality visualization is further enriched with information about the orientation in which the needle shall be inserted. Advantageously, the physician receives all the information about the planned pose of the needle to be inserted into the examination portion.

For calculating an end point of the needle, the knowledge about the length 1 of the needle is used. Then the needle end point $u_{CT}$ in the medical image data coordinate system is constructed at the start of the insertion by virtually moving the needle insertion point $q_{CT}$ in opposite direction of the planned needle direction. The planned needle direction $d_{CT}$ from the needle insertion point q to the planned needle end point is calculated as follows:

$$d_{CT} = (p_{CT} - q_{CT})/\|p_{CT} - q_{CT}\|, \qquad (4)$$

wherein $d_{CT}$ is normalized and $p_{CT}$ is the target needle tip position inside the body of a patient in the medical image data coordinate system.

The needle end point $u_{CT}$ is calculated as follows:

$$u_{CT} = q_{CT} - l\, d_{CT}, \qquad (5)$$

where $d_{CT}$ is normalized. This needle end point $u_{CT}$ is then also transformed to camera coordinates $u_{CAM}$ and preferably projected into the 2D camera image, wherein the needle end point $u_{2D}$ in the 2D camera image is calculated as follows:

$$u_{2D} = P_{CAM} u_{CAM} \quad . \qquad (6)$$

The needle insertion point q and the end point u are visualized for example as dots or as line ranging from $q_{2D}$ to $u_{2D}$ in a live camera image, preferably a 2D live camera image.

Hence, also preferred, the needle end point $u_{CT}$ at the start of the insertion is determined by virtually moving the needle insertion point $q_{CT}$ in the opposite direction of the planned needle direction, as explained in context with formula (4). Advantageously, a second marking point for positioning the needle at the start of the insertion is determined which facilitates the positioning and alignment of the needle at the beginning of the insertion.

As mentioned above, the information concerning an insertion pose of the needle in the received medical image data preferably comprises an end point of the needle head at the end of the needle intervention. Advantageously, the physician receives a precise information about the end position of the needle head he has to reach in the end of the insertion.

In case the information concerning a planned insertion pose comprises an end point of the needle head at the end of the needle intervention, preferably, the end point of the needle head at the end of the needle intervention is determined by adding the target needle tip position to the end point of the needle at the start of the insertion and subtracting therefrom the insertion point of the needle.

The end point $u_{CT}$ of the needle head (at the start of the intervention) is moved in needle direction to the end point $v_{CT}$ of the needle head at the end of the needle intervention:

$$v_{CT} = u_{CT} + p_{CT} - q_{CT}, \qquad (7)$$

wherein the needle tip is transferred to the target needle tip position $p_{CT}$ inside the body.

If the camera image data comprise 2D image data, a projection into the view plane of the camera is performed as explained above for determining a 2D projection $v_{2D}$ of the end point $v_{CT}$ of the needle head at the end of the needle intervention in the 2D image data:

$$v_{2D} = P_{CAM}T_{CT,CAM}v_{CT}. \qquad (8)$$

A visual indication like a dot is then placed at this position to indicate the stop position for the needle end point in the live 2D camera image.

Hence, if the camera image data comprises 2D camera image data, the step of determining the coordinates assigned to the insertion pose of the needle in the camera image data preferably includes the sub-steps of:

- transforming the coordinates $q_{CT}$, $u_{CT}$, $v_{CT}$ assigned to the insertion pose from the medical image data coordinate system into a 3D camera coordinate system,
- projecting the transformed coordinates $q_{CAM}$, $u_{CAM}$, $v_{CAM}$ from the 3D camera coordinate system into 2D camera image data, wherein 2D coordinates $q_{2D}$, $u_{2D}$, $v_{2D}$ in the 2D camera image data concerning the insertion pose are determined.

Advantageously, the target points of the needle are projected into 2D camera image data, which are used for observing the patient and in particular the examination portion.

Even if the camera image data comprises 3D camera image data, in the method for generating augmented camera image data for guiding a needle intervention according to the invention, the sub-step of projecting the coordinates concerning the planned insertion pose into the camera image data preferably comprises the step of applying a projection $T_{CT,CAM}$ to the coordinates concerning the insertion pose in the 3D camera coordinate system.

The invention is explained below with reference to the figures enclosed once again. The same components are provided with identical reference numbers in the various figures. The figures are usually not to scale.

FIG 1 shows a schematic illustration of an intervention using a needle guided by a laser beam according to prior art,

FIG 2 shows a schematic illustration of an intervention using a needle guided by a camera of a smartphone according to prior art,

FIG 3 shows a perspective view of a perspective projection of points in 3D-space onto an image plane,

FIG 4 shows a sectional view of a perspective projection of points in 3D-space onto the image view plane of a camera,

FIG 5 shows a 2D view of projected points in the image view plane of a camera,

FIG 6 shows a virtual image of a needle at the beginning of a treatment,

FIG 7 shows a virtual image of a needle, when the needle is partially inserted,

FIG 8 shows a virtual image of a needle including the planned needle end position,

FIG 9 shows a flow chart illustrating the camera-based method for guiding a needle intervention according to an embodiment of the invention,

FIG 10      shows a schematic view of an intervention control device according to an embodiment of the invention,

FIG 11      shows an intervention system according to an embodiment of the invention.

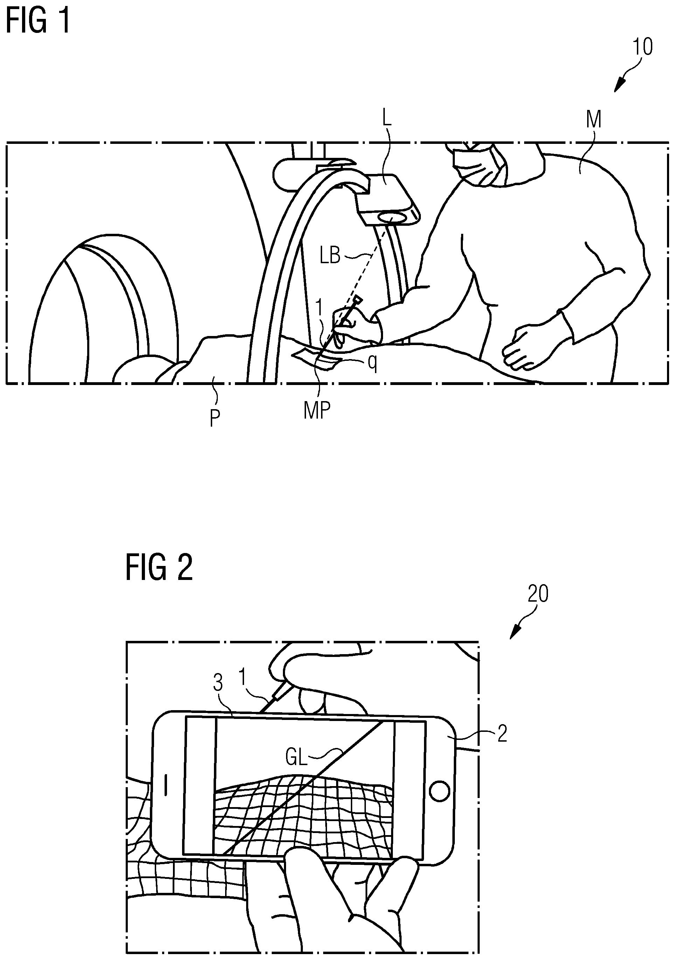

In FIG 1, a schematic illustration 10 of an intervention using a needle 1 guided by a laser beam LB of a laser L according to prior art is shown. As can be taken from FIG 1, the initial needle placement on a planned path is assisted by a laser L emitting a laser beam LB onto a patient P, wherein a marking point MP is projected onto the planned insertion position q of the needle 1. Hence, a physician M places the needle 1 on the marked insertion point q guided by the laser beam LB.

In FIG 2, a schematic illustration 20 of an intervention using a needle 1 guided by a camera of a smartphone 2 is depicted. A smartphone application overlays the planned angle on the smartphone's camera display 3 in real-time based on the smartphone's orientation. The needle's angle is selected by visually comparing the actual needle with the guideline GL in the display 3 of the smartphone 2. However, this system also lacks integration with the CT system.

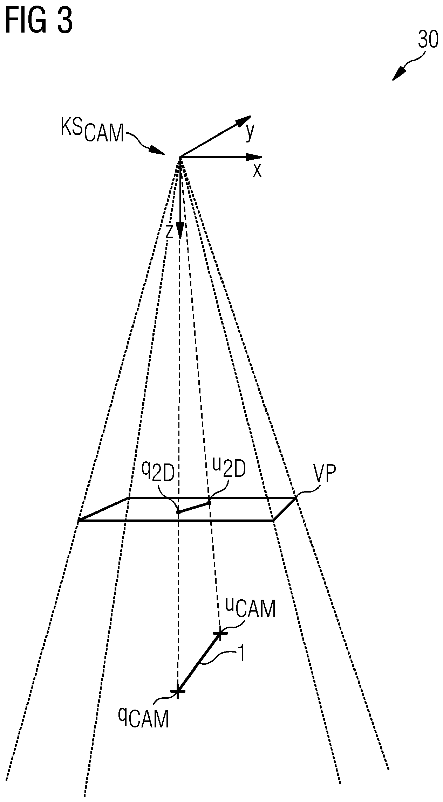

In FIG 3, a perspective view 30 of a perspective projection of points q, u in 3D-space onto an image plane or view plane VP is shown. In the upper portion of FIG 3 a coordinate system $KS_{CAM}$ of the camera is depicted, wherein the origin of the coordinate system represents the camera origin. Further, in the view plane VP of the camera a projection of the needle 1 and particularly the projected insertion point $q_{2D}$ and the projected end point $u_{2D}$ of the needle 1 are illustrated. Further, on the lower part of FIG 3 the needle 1 itself in 3D space is illustrated and the insertion point $q_{CAM}$ and the endpoint $u_{CAM}$ of the needle 1 at the start of the intervention are depicted.

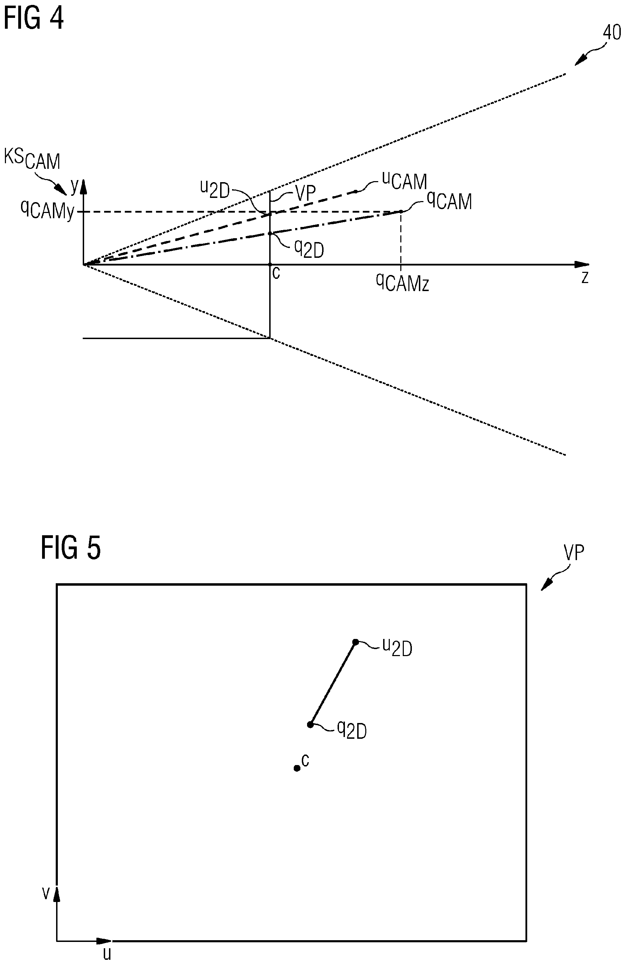

In FIG 4, a sectional view 40 of a perspective projection of points in 3D-space onto the image view plane VP is shown. As can be taken from the radiation theorem, the v-coordinate $q_{2Dv}$ of the projection $q_{2D}$ of the insertion point is achieved by:

$$q_{2Dv} = f_y \ * \ q_{CAMy} \ / \ q_{CAMz} \ + \ c_v, \qquad (9)$$

wherein $f_y$ is the focal length in y-direction, $q_{CAMy}$ is the y-coordinate of the insertion point $q_{CAM}$ in the 3D camera coordinate system $KS_{CAM}$, $q_{CAMz}$ is the z-coordinate of the insertion point $q_{CAM}$ in the 3D camera coordinate system $KS_{CAM}$, $c_v$ is the v-coordinate of the intersection point c of the z axis of the 3D camera coordinate system with the view plane VP of the camera. In an analogue manner, also the v-coordinate $u_{2Dv}$ of the projection $u_{2D}$ of the end point u at the start of an intervention can be calculated.

Analogue to formular (9) also the u-coordinate $q_{2Du}$ of the projection $q_{2D}$ of the insertion point q can be calculated as follows:

$$q_{2Du} = f_x \ * \ q_{CAMx} \ / \ q_{CAMz} \ + \ c_u, \qquad (10).$$

wherein $f_x$ is the focal length in x-direction, $q_{CAMx}$ is the x-coordinate of the insertion point $q_{CAM}$ in the 3D camera coordinate system $KS_{CAM}$, $q_{CAMz}$ is the z-coordinate of the insertion point $q_{CAM}$ in the 3D camera coordinate system $KS_{CAM}$, $c_u$ is the u-coordinate of the intersection point c of the z axis of the 3D camera coordinate system with the view plane VP of the camera.

In FIG 5, a 2D view of the projected insertion point $q_{2D}$ and the projected end point $u_{2D}$ at the start of the intervention in the image plane VP is illustrated. As can be taken from FIG 5, in the center of the view plane VP, the intersection point c of the z axis of the 3D camera coordinate system with the view plane VP of the camera is marked. Further the u-axis u and the v-axis v of the coordinate system of the view plane VP is marked on the lower left portion of FIG 5.

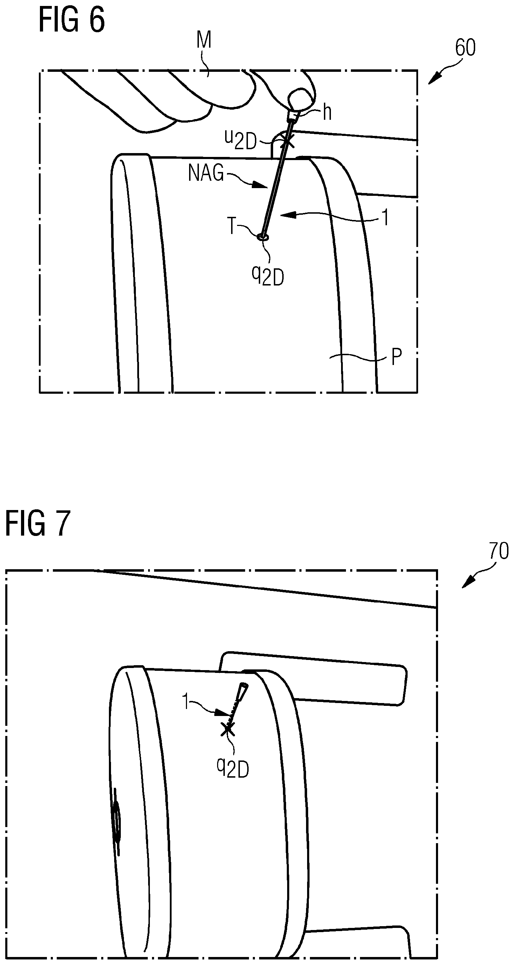

In FIG 6, a virtual image 60 of a needle 1 at the beginning of a treatment is shown. The needle alignment guide NAG, i.e. the initial needle orientation, is drawn as a solid line. The user needs to align the real needle tip T with one ending of the virtual guidance needle overlay and the needle head h with the other ending of the virtual needle.

The visualization $q_{2D}$ of the needle insertion point q is achieved as follows: in order to provide augmented-reality guidance to the physician M during the intervention, a real-time view of the patient P acquired with the camera is displayed on a screen, for example on an in-room monitor or a tablet next to the patient P. In addition, the planned needle insertion point q is displayed as an overlay graphic, as illustrated in FIG 6. Therefore, the transformation $T_{CT,AM}$ from scanner / medical image coordinates to camera coordinates is determined in an extrinsic calibration step. This step is performed once during installation of the camera. The calibration can be performed for example with a checkerboard phantom that is aligned to the scanner positioning lasers.

Assuming that the patient P has not moved since the planning scan was performed, the insertion point $q_{CT}$ from the planning scan can be transferred to the camera image point $q_{CAM}$ using the scanner to camera transform $T_{CT,CAM}$ as mentioned in formula (1).

In case at the intervention treatment that table position is different to the planning scan position, an additional translation regarding the new table position (described by the transformation $T_{TABLE}$) has to be applied while computing the point in camera coordinates according to formula (2).

The needle insertion point in camera coordinates $q_{CAM}$ is then projected into the camera image using the intrinsic calibration expressed through the projection $P_{CAM}$ according to formula (3).

The intrinsic calibration of the camera consists of the focal length f, the principal point, and distortion coefficients. A visual marker like a dot is displayed as an overlay onto the live camera image. The physician can then align the tip of the actual needle with the marker to insert the needle at the right position.

In FIG 7, a virtual image 70 of a needle 1 when the needle 1 is partially inserted, is shown. The projected insertion point $q_{2D}$ is visualized as a cross.

The augmented-reality visualization may be further enriched with information about the orientation in which the needle 1 shall be inserted. Therefore, the length l of the needle 1 is entered into the system. The system then constructs the needle end point $u_{CT}$ at the start of the insertion by moving the needle insertion point $q_{CT}$ in opposite direction of the planned needle direction. The direction $d_{CT}$ from the needle insertion point q to the planned needle end point is calculated according to formula (4).

The needle end point $u_{CT}$ is calculated according to formula (5) .

This needle end point $u_{CT}$ is then also transformed to camera coordinates $u_{CAM}$ and projected into the 2D camera image according to formula (6).

The needle insertion point q and end point u are visualized for example as dots or as line ranging from $q_{2D}$ to $u_{2D}$ in the live 2D camera image, as illustrated in FIG 6. At the start of the intervention, the physician aligns the tip T of the actual needle 1 in the live 2D camera image with the projected needle insertion point $q_{2D}$ to set the insertion point and the end of the needle with the projected needle end point $u_{2D}$ to set the needle direction.

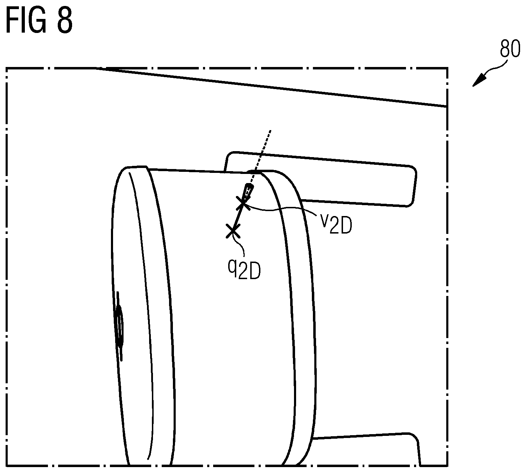

In FIG 8, a virtual image 80 of a needle 1 including the planned needle end position is shown. The planned needle end position v is visualized by the projected planned needle end position $V_{2D}$ to indicate to the user when to stop the insertion as the planned target position p of the tip of the needle (not shown) is reached. Further, also the projected insertion point $q_{2D}$ is visualized in FIG 8. The initial needle orientation is drawn as a dashed line.

The visualization may also comprise information about the target point p where the needle shall stop inside the body. The tip of the needle obviously cannot be seen as it approaches the target point p inside the body. However, the visualization on the outside of the body can indicate where the end of the needle shall be when the tip is at the target. Therefore, the end point $v_{CT}$ of the needle head (at the start of the intervention) is moved in needle direction according to formula (7). Then, the position $v_{2D}$ of the end point $v_{CT}$ of the needle head at the end of the intervention in 2D camera image data is calculated according to formula (8). A visual indication like a dot is then placed at this position to indicate the stop position for the needle end point in the live 2D camera image, as illustrated in FIG 8.

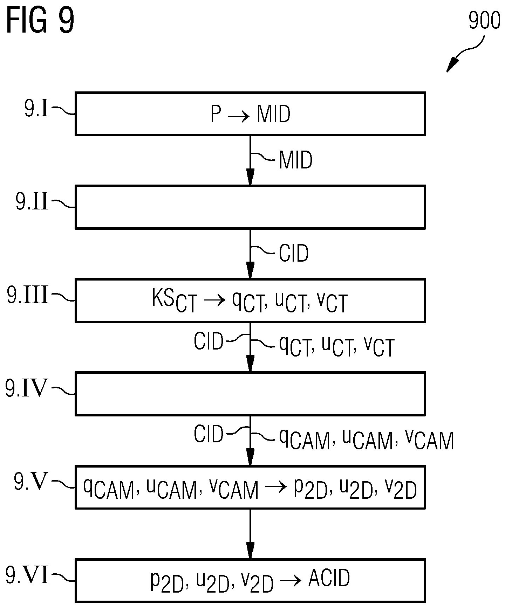

In FIG 9, a flow chart 900 is shown, illustrating the camera-based method for determining information for guiding a needle intervention.

In step 9.I, medical image data MID of an examination portion of a patient P are received.

In step 9.II, camera image data CID of the examination portion are received.

In step 9.III, an planned insertion position $q_{CT}$, a needle end point $u_{CT}$ during the start of insertion of the needle 1 and a planned needle end position $v_{CT}$ are determined in the received medical image data MID in the medical image data coordinate system $KS_{CT}$. As mentioned above, the template for the planned insertion of a needle is preferably determined such that the violation of organs is avoided and the examination portion is hit by the needle.

In step 9.IV, the coordinates $q_{CT}$, $u_{CT}$, $v_{CT}$ assigned to the planned insertion pose of the needle 1 are transformed from the medical image data coordinate system $KS_{CT}$ into a 3D camera coordinate system $KS_{CAM}$.

In step 9.V, the transformed coordinates $q_{CAM}$, $u_{CAM}$, $v_{cam}$ are projected into a 2D camera image coordinate system and 2D coordinates $p_{2D}$, $u_{2D}$, $v_{2D}$ assigned to the planned insertion pose are determined.

In step 9.VI, augmented camera image data ACID are generated based on the determined 2D coordinates $p_{2D}$, $u_{2D}$, $v_{2D}$ and the camera image data CID.

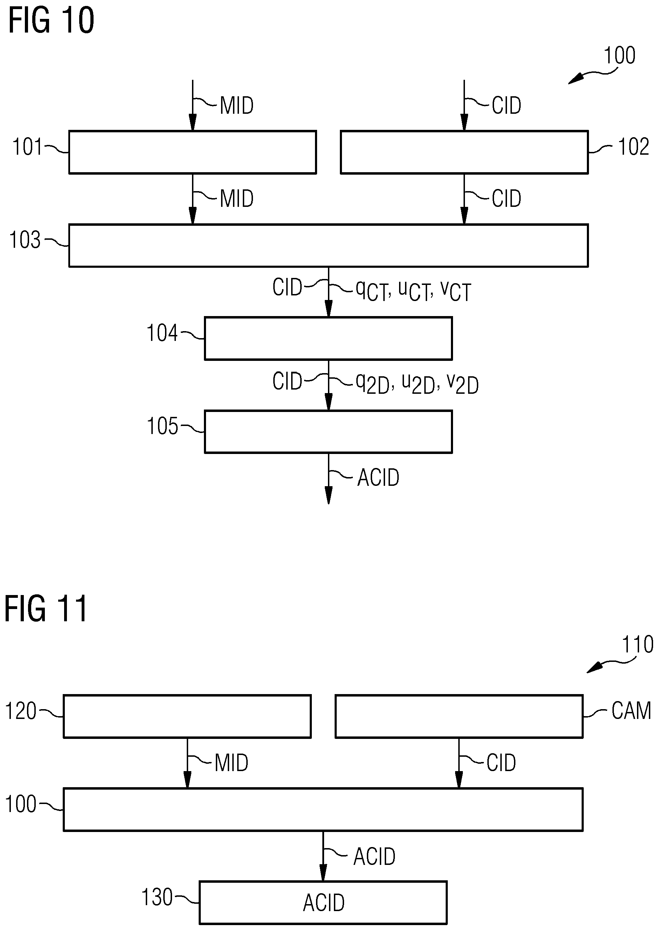

In FIG 10, a schematic view of an intervention control device 100 according to an embodiment of the invention is shown.

The intervention control device 100 comprises a medical image data interface 101 for receiving medical image data MID of an examination portion of a patient.

Further, the intervention control device 100 comprises a camera image data interface 102 for receiving camera image data CID of the examination portion.

Furthermore, the intervention control device 100 comprises a determination unit 103 for determining information concerning an insertion pose of the needle 1 in the received medical image data MID, wherein coordinates $q_{CT}$, $u_{CT}$, $v_{CT}$ assigned to the insertion pose of the needle 1 in the medical image data coordinate system $KS_{CT}$ are determined.

The intervention control device 100 also includes a transformation unit 104 for transforming the coordinates $q_{CT}$, $u_{CT}$, $v_{CT}$ assigned to the insertion pose of the needle 1 in the medical image data coordinate system $KS_{CT}$ into 2D coordinates $q_{2D}$, $u_{2D}$, $v_{2D}$ assigned to a 2D view plane of the camera.

The intervention control device 100 further comprises a generation unit 105 for generating augmented camera image data ACID based on the determined coordinates $q_{2D}$, $u_{2D}$, $v_{2D}$ in the camera coordinate system $KS_{CAM}$ and based on the received camera image data CID.

In FIG 11, an intervention system 110 comprising a medical imaging system 120 is shown. For example, the medical imaging system 120 comprises a CT system. The medical imaging system 120 is used for generating medical image data MID from an examination portion of a patient. It is planned to insert a needle into an insertion portion and further to an examination portion of a patient for carrying out an intervention in the examination portion. For identifying and localizing the insertion portion and the examination portion, the medical image data MID are used. Further, the intervention system 110 comprises an observation camera CAM. The camera CAM is used for observing the patient and in particular the insertion portion by recording camera image data CID. The intervention system 110 also comprises a needle 1 for intervention (not shown in FIG 11). Furthermore, the intervention system 110 comprises an intervention control device 100 as illustrated in FIG 10 for controlling the intervention using the needle 1. The intervention is performed based on augmented camera image data generated by the intervention control device 100.

Further, the intervention system 110 comprises a display 130 for displaying the augmented camera image data ACID the needle 1 and for displaying a planned pose of the needle 1 based on determined coordinates $p_{2D}$, $u_{2D}$, $v_{2D}$ provided by the intervention control device 100.

Finally, it should be pointed out once again that the detailed methods and structures described above are exemplary embodiments and that the basic principle can also be varied in wide areas by the person skilled in the art without leaving the scope of the invention, insofar as it is specified by the claims. For the sake of completeness, it should also be noted that the use of the indefinite articles "a" or "an" does not exclude the fact that the characteristics in question can be present multiple times. Likewise, the term "unit" does not exclude the fact that it consists of several components, which may also be spatially distributed. Further, independent of the grammatical term usage, individuals with male, female or other gender identities are included within the term.

**Claims**

1. Computer-implemented method for generating augmented camera image data (ACID) for guiding a needle intervention,

   - receiving medical image data (MID) of an examination portion of a patient (P),
   - receiving camera image data (CID) of the examination portion acquired using a camera (CAM),
   - determining information concerning a planned insertion pose of the needle (1) based on the received medical image data (MID), wherein coordinates ($q_{CT}$, $u_{CT}$, $v_{CT}$) assigned to the planned insertion pose of the needle (1) in a medical image data coordinate system ($KS_{CT}$) are determined,
   - determining coordinates ($q_{2D}$, $u_{2D}$, $v_{2D}$, $q_{CAM}$, $u_{CAM}$, $v_{CAM}$) assigned to the planned insertion pose of the needle (1) in a camera coordinate system ($KS_{CAM}$) of the camera (CAM) by transforming the coordinates ($q_{CT}$, $u_{CT}$, $v_{CT}$) assigned to the planned insertion pose in the medical image data coordinate system ($KS_{CT}$) into the camera coordinate system ($KS_{CAM}$),
   - generating augmented camera image data (ACID) based on the determined coordinates ($q_{2D}$, $u_{2D}$, $v_{2D}$, $q_{CAM}$, $u_{CAM}$, $v_{CAM}$) in the camera coordinate system ($KS_{CAM}$) and based on the received camera image data (CID).

2. Method according to claim 1, wherein the information concerning the planned insertion pose of the needle (1) in the received medical image data (MID) comprises a needle insertion point ($q_{CT}$).

3. Method according to claim 2, wherein the information concerning the planned insertion pose of the needle (1) in the received medical image data (MID) comprises a needle orientation for the needle intervention and the step of determining information concerning the planned insertion pose of the needle (1) in the received medical image data (MID) comprises the sub-step of determining at least one of:

   - an end point ($u_{CT}$) of the needle at the start of the insertion based on a target needle tip position ($p_{CT}$),
   - a planned needle direction ($d_{CT}$)
   and
   - the length (I) of the needle.

4. Method according to claim 3, wherein the end point ($u_{CT}$) of the needle at the start of the insertion is determined by virtually moving the needle insertion point ($q_{CT}$) in the opposite direction of the planned needle direction ($d_{CT}$).

5. Method according to any of the preceding claims, wherein the information concerning the planned insertion pose of the needle (1) in the received medical image data (MID) comprises a planned end point ($v_{CT}$) of the needle head at the end of the needle intervention.

6. Method according to claim 5, wherein the planned end point ($v_{CT}$) of the needle head at the end of the needle intervention is determined by addition of the target needle tip position ($p_{CT}$) to the end point ($u_{CT}$) of the needle at the start of the insertion and subtracting therefrom the needle insertion point ($q_{CT}$).

7. Method according to any of the preceding claims, wherein the camera image data (CID) comprise 2D camera image data and the step of determining the coordinates ($q_{2D}$, $u_{2D}$, $v_{2D}$, $q_{CAM}$, $u_{CAM}$, $v_{CAM}$) assigned to the planned insertion pose of the needle (1) in the camera coordinate system ($KS_{CAM}$) of the camera (CAM) includes the sub-steps of:

- transforming the coordinates ($q_{CT}$, $u_{CT}$, $v_{CT}$) assigned to the planned insertion pose from the medical image data coordinate system ($KS_{CT}$) into a 3D camera coordinate system ($KS_{CAM}$),
- projecting the transformed coordinates ($q_{CAM}$, $u_{CAM}$, $v_{CAM}$) into 2D camera image data, wherein 2D coordinates ($p_{2D}$, $u_{2D}$, $v_{2D}$) assigned to the planned insertion pose are determined.

8. Method according to claim 7, wherein the sub-step of transforming the coordinates ($q_{CT}$, $u_{CT}$, $v_{CT}$) assigned to the planned insertion pose from the medical image data coordinate system ($KS_{CT}$) into a 3D camera coordinate system ($KS_{CAM}$) comprises an application of a camera transform ($T_{CT,CAM}$) to the coordinates ($q_{CT}$, $u_{CT}$, $v_{CT}$) assigned to the planned insertion pose in the medical image data coordinate system ($KS_{CT}$).

9. Method according to claim 7 or 8, wherein the sub-step of transforming the coordinates ($q_{CT}$, $u_{CT}$, $v_{CT}$) assigned to the planned insertion pose from the medical image data coordinate system ($KS_{CT}$) into a 3D camera coordinate system ($KS_{CAM}$) comprises an application of a transformation ($T_{TABLE}$) representing a new table position.

10. Method according to claim 8 or 9, wherein the camera transform ($T_{CT,CAM}$) is determined based on an extrinsic calibration step.

11. Camera-based method for guiding a needle intervention, comprising the steps:

- generating augmented camera image data (ACID) by performing the method according to any of the claims 1 to 10,
- displaying the augmented camera image data (ACID).

12. Intervention control device (100), comprising:

- a medical image data interface (101) for receiving medical image data (MID) of an examination portion of a patient,
- a camera image data interface (102) for receiving camera image data (CID) of the examination portion,
- a determination unit (103) for determining information concerning a planned insertion pose of the needle (1) based on the received medical image data (MID), wherein coordinates ($q_{CT}$, $u_{CT}$, $v_{CT}$) assigned to the planned insertion pose of the needle (1) in a medical image data coordinate system ($KS_{CT}$) are determined,
- a transformation unit (104) for determining coordinates ($q_{2D}$, $u_{2D}$, $v_{2D}$, $q_{CAM}$, $u_{CAM}$, $v_{CAM}$) assigned to the planned insertion pose (P) of the needle (1) in a camera coordinate system ($KS_{CAM}$) of the camera (CAM) by transforming the coordinates ($q_{CT}$, $u_{CT}$, $v_{CT}$) assigned to the planned insertion pose from the medical image data coordinate system ($KS_{CT}$) into the camera coordinate system ($KS_{CAM}$),
- a generation unit (105) for generating augmented camera image data (ACID) based on the determined coordinates ($q_{2D}$, $u_{2D}$, $v_{2D}$, $q_{CAM}$, $u_{CAM}$, $v_{CAM}$) in the camera coordinate system ($KS_{CAM}$) and based on the received camera image data (CID).

13. Intervention system (110), comprising:

- a medical imaging system (120),
- a camera (CAM),
- a needle (1) for an intervention,
- an intervention control device (100) according to claim 12 for generating augmented camera image data (ACID) based on medical image data (MID) from the medical imaging system (120) and camera image data (CID) from the camera (CAM) for controlling the intervention using the needle (1) based on the augmented camera image data (ACID),
- a display (130) for displaying the augmented camera image data (ACID) generated by the intervention control device (100) .

14. A computer program product comprising instructions which, when the program is executed by a computer, cause the computer to carry out the steps of the method of any of the claims 1 to 11.

15. A computer-readable storage medium comprising instructions which, when executed by a computer, cause the computer to carry out the steps of the method of any of the claims 1 to 11.

FIG 1

FIG 2

FIG 3

30

$KS_{CAM}$

y

x

z

$q_{2D}$  $u_{2D}$  VP

$u_{CAM}$

1

$q_{CAM}$

## FIG 4

## FIG 5

## FIG 6

## FIG 7

# FIG 8

## FIG 9

900

9.I $\quad$ $P \rightarrow MID$

MID

9.II

CID

9.III $\quad$ $KS_{CT} \rightarrow q_{CT}, u_{CT}, v_{CT}$

CID $\quad q_{CT}, u_{CT}, v_{CT}$

9.IV

CID $\quad q_{CAM}, u_{CAM}, v_{CAM}$

9.V $\quad$ $q_{CAM}, u_{CAM}, v_{CAM} \rightarrow p_{2D}, u_{2D}, v_{2D}$

9.VI $\quad$ $p_{2D}, u_{2D}, v_{2D} \rightarrow ACID$

## FIG 10

100

MID

101

CID

102

MID

CID

103

CID — $q_{CT}$, $u_{CT}$, $v_{CT}$

104

CID — $q_{2D}$, $u_{2D}$, $v_{2D}$

105

ACID

## FIG 11

110

120

CAM

MID

CID

100

ACID

130

ACID

Europäisches
Patentamt

European
Patent Office

Office européen
des brevets

# EUROPEAN SEARCH REPORT

**Application Number**

EP 24 15 3415

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/409290 A1 (KRÜGER TIMO [DE]) 29 December 2022 (2022-12-29) * paragraph [0032] - paragraph [0042] * ----- | 1-15 | INV. A61B90/36 A61B34/10 |
| X | US 2021/153969 A1 (COHN WILLIAM [US] ET AL) 27 May 2021 (2021-05-27) * column 50; figure 7 * ----- | 1,11-15 | ADD. A61B90/00 |
| X | WO 2023/086592 A2 (MAT NV [BE]; MAT USA LLC [US]) 19 May 2023 (2023-05-19) * paragraphs [0245], [0287] * ----- | 1,11-15 | |
| X | US 9 498 132 B2 (DEUTSCHES KREBSFORSCHUNGSZENTRUM STIFTUNG DES ÖFFENTLICHEN RECHTS [DE]) 22 November 2016 (2016-11-22) * figure 3 * ----- | 1,11-15 | |

**TECHNICAL FIELDS SEARCHED (IPC)**

A61B

The present search report has been drawn up for all claims

| Place of search | Date of completion of the search | Examiner |
|---|---|---|
| Munich | 18 June 2024 | Franz, Volker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 3415

This annex lists the patent family members relating to the patent documents cited in  the above-mentioned European search report.
The members are as contained in the European Patent Office EDP file on
The European Patent Office is in no way liable for these particulars which are merely given for the purpose of information.

18-06-2024

| Patent document cited in search report | Publication date | Patent family member(s) | Publication date |
|---|---|---|---|
| US 2022409290 A1 | 29-12-2022 | CN 114727845 A | 08-07-2022 |
| | | DE 102019130368 A1 | 12-05-2021 |
| | | EP 4057928 A1 | 21-09-2022 |
| | | JP 2023502172 A | 20-01-2023 |
| | | KR 20220100613 A | 15-07-2022 |
| | | US 2022409290 A1 | 29-12-2022 |
| | | WO 2021094354 A1 | 20-05-2021 |
| US 2021153969 A1 | 27-05-2021 | AU 2020392972 A1 | 14-07-2022 |
| | | BR 112022010001 A2 | 16-08-2022 |
| | | CA 3162550 A1 | 03-06-2021 |
| | | CN 114760951 A | 15-07-2022 |
| | | EP 4065031 A1 | 05-10-2022 |
| | | IL 293279 A | 01-07-2022 |
| | | JP 2023503603 A | 31-01-2023 |
| | | KR 20220105662 A | 27-07-2022 |
| | | US 2021153969 A1 | 27-05-2021 |
| | | WO 2021105785 A1 | 03-06-2021 |
| WO 2023086592 A2 | 19-05-2023 | AU 2022384283 A1 | 30-05-2024 |
| | | WO 2023086592 A2 | 19-05-2023 |
| US 9498132 B2 | 22-11-2016 | EP 2452649 A1 | 16-05-2012 |
| | | EP 2637593 A1 | 18-09-2013 |
| | | US 2013245461 A1 | 19-09-2013 |
| | | WO 2012062482 A1 | 18-05-2012 |

EPO FORM P0459

For more details about this annex : see Official Journal of the European Patent Office, No. 12/82